(19) Europäisches Patentamt

European Patent Office

Office européen des brevets

(11) Publication number: **0 256 683 A2**

# (12) EUROPEAN PATENT APPLICATION

(21) Application number: 87306467.9

(22) Date of filing: 22.07.87

(51) Int. Cl.⁴: **A61M 25/00**

(30) Priority: 04.08.86 US 893354

(43) Date of publication of application:
24.02.88 Bulletin 88/08

(84) Designated Contracting States:
AT BE CH DE ES FR GB GR IT LI LU NL SE

(71) Applicant: **ARIES MEDICAL INCORPORATED**

**Wilmington Delaware(US)**

(72) Inventor: **Neuman, Harold L.**
**35 Colonial Drive**
**Reading Massachusetts 01867(US)**
Inventor: **Lombardi, Edward J.**
**143 Oakland Street**
**Malden Massachusetts 02148(US)**

(74) Representative: **Harland, Linda Jane**
**c/o Reddie & Grose 16 Theobalds Road**
**London WC1X 8PL(GB)**

(54) Means for furling a balloon of a balloon catheter.

(57) A packaged deflated balloon catheter includes a catheter (12) coupled to a balloon (10) which is received in a furling device (36). The package comprises a tray (26) provided with recesses (28,30,32), for receiving the furling device (36), a holding sheath (38) and the catheter (12). The furling device (36) comprises means for retaining the walls of the balloon (10) on either side of its longitudinal axis flat so that relative rotation of the means for retaining and the balloon causes the balloon to be furled. The furled balloon (10) can then be withdrawn from the furling device (36) into the sheath (38) which retains it in a furled condition for insertion. The catheter may also include a syringe (18) connected to the catheter (12) by means of which the deflated balloon (10) can be evacuated prior to furling.

FIG. 3 FIG. 3B FIG. 3C FIG. 3A FIG. 3G

EP 0 256 683 A2

## Means for Furling a Balloon of a Balloon Catheter

This invention relates to balloon catheters such as the intra-aortic balloon (IAB) catheter, and particularly to methods for wrapping the balloon prior to insertion into a patient.

Intra-aortic balloon counterpulsation (IABC) has been in use widely for several years. Several attempts have been made over the years to improve IAB catheters and insertion techniques in order to enable safer and more rapid insertion so that the IAB can be more effectively used.

Among the reported complications of IABC in general and the balloon catheters in particular are:

1. A large diameter percutaneous introducer sheath is required in order to insert the wrapped IAB, resulting in difficult hemostasis, particularly upon removal by percutaneous technique, and potential thrombus formation between the lumen of the introducer and the IAB catheter.

2. Impaired blood flow and subsequent limb ischemia distal to the IAB insertion site, sometimes requiring surgical amputation.

3. Potential over-wrapping of the balloon and concomitant damage to the balloon membrane.

4. Occasional failure of mechanically wrapped IABs to completely unwrap once in the patient, leading to impaired counterpulsation and thrombus formation in the folds of the wrapped balloon, potentially embolizing.

Initially, the balloon was wrapped or furled by hand by rolling the two balloon flaps around the central support. Although the tightness of this roll correlated with the ease of insertion, the furling was inconsistent.

Some mechanical devices were utilized to twist the balloon around its center support to facilitate insertion. Such devices have been described in United States Patents Nos. 4,531,512; 4,261,339; 4,422,447; 4,444,186; and 4,362,150. However, these devices provide potential for over-wrapping the balloon with resulting damage to the balloon membrane or for incomplete wrapping of the balloon after insertion, leading to impaired counterpulsation and thrombus formation.

It has also been proposed to pre-wrap the balloon prior to packaging and shipping. However, when packaged in a wrapped condition for a length of time prior to use, the collapsed walls of the balloon resulting from being wrapped for prolonged periods of time tend to adhere to each other and the kinks tend to persist so that, when used, the balloon does not expand to its full capacity and provides sites for thrombus formation. It is the purpose of the invention to try to overcome these various difficulties by providing for the packaging of unfurled balloons and mechanical furling immediately prior to use.

In accordance with a first aspect of the invention, there is provided a structure for furling a deflated, generally flat balloon of a balloon catheter and confining at least a portion thereof within a sheath preparatory to use, said structure comprising relatively rotatable means engaged with the ends of the deflated balloon and with the balloon intermediate its ends, said means being relatively rotatable to furl the balloon about its longitudinal axis in opposite directions with respect to its mid-length to contract the deflated balloon to a less than predetermined diameter from end-to-end, a sheath defining an elongate tubular passage corresponding in internal diameter to the outside diameter of the furled balloon, said sheath supported in axial alignment with the furled balloon, and means for effecting movement of a portion at least of the furled balloon into said sheath to constrain the furled balloon in said furled condition.

In a further aspect the invention provides a packaged, deflated balloon catheter, including a catheter connected at its distal end to the proximal end of a balloon, a check valve connected to the proximal end of the catheter and a wire connected at its proximal end to the proximal end of the catheter end and its distal end to the distal end of the balloon, said package comprising a tray containing a recess for receiving the deflated balloon, catheter and check valve, a furling device non-rotatably disposed in the recess about the deflated balloon and a holding sheath disposed in the recess adjacent the furling device, said catheter and wire being relatively rotatable with respect to the mid-section of said balloon and to the furling device to furl the balloon within the furling device from its ends towards its mid-section.

In a third aspect the invention provides a furling fixture for furling a deflated balloon of a balloon catheter, said fixture comprising an elongate retaining structure defining a recess for retaining structure defining a recess corresponding substantially in width to the width of the deflated balloon and in minimum depth to substantially the thickness of the deflated balloon, said fixture being operable when rotated relative to the ends of the balloon catheter to furl the balloon from its mid-length to its ends to no greater than a predetermined diameter from end to end.

In a fourth aspect the invention provides a method of preparing a balloon catheter for insertion into a patient, said method comprising evacuating the balloon to cause it to become flattened throughout its length from end-to-end and to eliminate air pockets, furling the flattened balloon about its longitudinal axis commencing at its mid-length and proceeding to its opposite ends to condense the balloon to a predetermined diameter from end-to-end and confining the furled, condensed balloon within a tubular sheath of an inside diameter corresponding to substantially the predetermined diameter of the furled balloon, wherein said furling step is accomplished prior to removing the balloon catheter from a tray by using a furling fixture disposed within said tray, said fixtures comprising means for retaining the opposite broad faces of the deflated balloon in a substantially flat position at opposite sides of the longitudinal axis, said means being operable to effect rotation of the balloon at opposite sides of its longitudinal axis relative to its longitudinal axis and simultaneously to relinquish the balloon at opposite sides of its longitudinal axis for constriction there to a furled diameter of substantially uniform cross section from end-to-end.

Thus, an apparatus embodying aspects of the invention includes means for effecting rotation of a deflated balloon of a balloon catheter intermediate its ends relative to its ends to furl the balloon in opposite directions from its mid-length to its ends to a predetermined diameter, means for evacuating the balloon and means for receiving and retaining the furled evacuated balloon preparatory to use. The means for effecting furling may comprise a furling device in which the deflated balloon is disposed intermediate its ends, a catheter connected at one end to one end of the balloon, a stiff wire (i.e. a guide or stylet wire which is used to insert the IAB into a patient and then removed) connected at one end to the distal end of the balloon and means for rotating the catheter and wire in unison relative to the furling device. The furling device may comprise a structure embodying a recess defined by spaced, parallel surface of a transverse width corresponding substantially to the width of the deflated balloon and in minimum depth to the thickness of the deflated balloon medially of which there is a groove of a cross section corresponding to the desired furled diameter of the balloon. The means for receiving and retaining the furled balloon comprises a sheath consisting of a tube-like structure having an inner diameter at least equal to the diameter of the furled balloon. In one embodiment, the sheath comprises confronting structures articulated along one longitudinal side and detachably interengaged along their other sides, said structures containing in their confronting sides longitudinal grooves of half circular cross section which collectively define a longitudinal groove of circular cross section. The invention may further provide a package structure comprising a recessed tray within which is disposed the deflated balloon, the catheter, the coupling and a syringe together with the furling device positioned about the deflated balloon and the retaining sheath disposed about the catheter. A cover is removably applied to the tray and, when removed, enables access to the syringe and coupling for evacuating the balloon, furling the balloon and withdrawing the furled balloon into the retaining sheath.

A preferred method in accordance with the invention comprises the steps of evacuating the balloon and, furling the evacuated balloon from its mid-length in opposite directions to its opposite ends by means of a furling device and drawing the furled balloon into a retaining sheath. Furling may be achieved by supporting the furled balloon between opposed friction-engendering surfaces, longitudinally of which there is a groove corresponding in cross section to the diameter of the furled balloon and rotating the ends of the balloon in a direction to contract the balloon from the friction-engendering surfaces into the groove.

Two embodiments of the invention will now be described in detail, by way of example only, with reference to the drawings, wherein:

FIG. 1 is a plan view of a percutaneous balloon, attached at one end to a catheter, to the opposite end of which is connected by means of a coupling a syringe and showing a wire extending from the coupling through the catheter and balloon to the distal end of the balloon;

FIG. 2 is a fragmentary view showing the balloon partially furled;

FIG. 3 is a plan view of a package with the cover broken away to show the disposition of the balloon, catheter, coupling and syringe therein;

FIG. 3A is a section taken on the line 3A-3A of FIG. 3;

FIG. 3B is a section taken on the line 3B-3B of FIG. 3;

FIG. 3C is a section taken on the line 3C-3C of FIG. 3;

FIG. 3D is a section taken on the line 3D-3D of FIG. 3;

FIG. 4 is a plan view to larger scale partially broken away of the furling device;

FIG. 5 is a transverse section taken on the line 5-5 of FIG. 4 without the balloon;

FIG. 6 is a transverse section taken on the line 6-6 of FIG. 4 showing a balloon in the furling cavity;

FIG. 7 is an elevation of one embodiment of a retaining sheath;

FIG. 8. is an elevation of the retaining sheath of FIG. 7 opened out flat;

FIG. 9 is an elevation of the balloon furled and partially constrained within the restraining sheath;

FIG. 10 is a plan view of an alternative form of package with the cover broken away to show the disposition of the balloon, catheter, coupling and syringe;

FIG. 11 is a view similar to FIG. 10 showing an alternative disposition of the restraining sheath;

FIG. 12 is a section taken on the line 12-12 of FIG. 10; and

FIG. 13 is a section taken on the line 13-13 of FIG. 10.

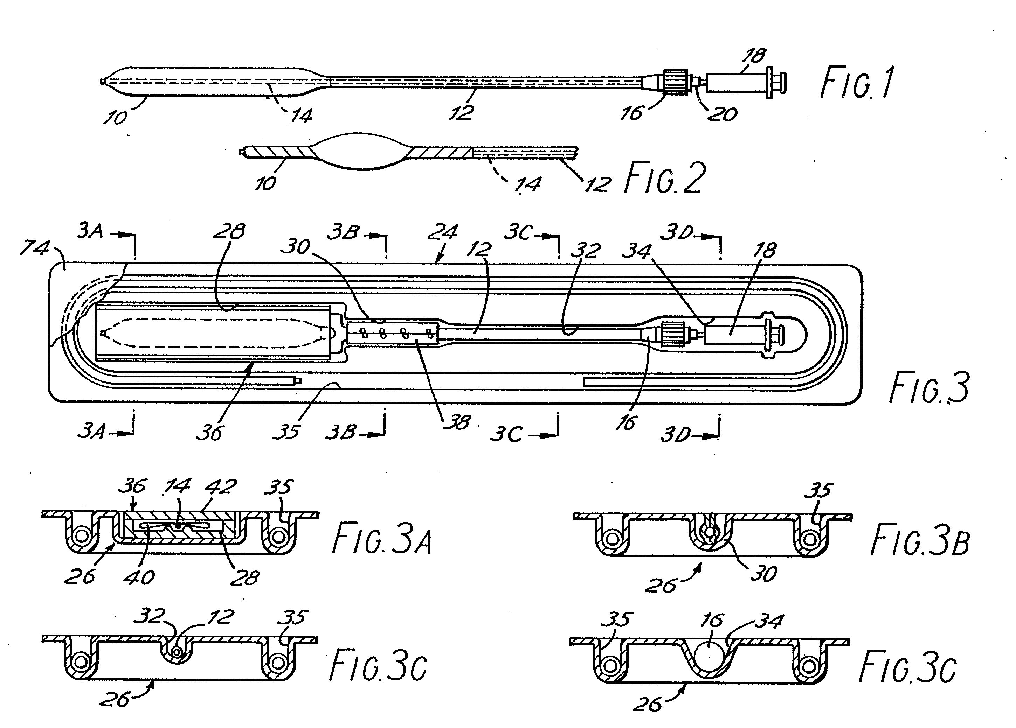

Referring to FIG. 1, there is shown a percutaneous balloon 10, a balloon catheter 12 connected at one end to one end of the balloon, a wire 14 connected at one end to the other end of the balloon and extending longitudinally through the balloon and through the catheter and a coupling 16 connecting the other end of the catheter to the other end of the wire. The coupling 16 is for connecting the catheter and, hence, the balloon to a syringe 18 or vacuum pump (not shown) for the purpose of evacuating the balloon and is desirably provided with a check valve 20 for maintaining the vacuum once the balloon has been evacuated. The coupling 16 further provides for connecting the catheter and balloon to one end of a tube, the other end of which may be connected to a diastolic pump, not shown, for the purpose of alternately inflating and deflating the balloon after it is inserted into a patient.

In accordance with the invention, there is provided a package 24, FIG. 3, for the aforesaid structure in the form of a tray 26 which is designed to contain the structure prior to use with the balloon deflated and to provide for furling the balloon incident to its use. It is a purpose of this invention to package the balloon in such a way as to eliminate the disadvantage of a pre-furled balloon which, if furled for any length of time within the package, can resist unfurling, and which retains crease lines after unfurling which tend to obstruct flow and cause incomplete expansion. It is also a purpose of this invention to provide a means for conventionally furling an IAB balloon so that once inserted into a patient, it automatically unfurls, having no twists in the balloon that could cause coagulation.

As herein illustrated in one form, the package comprises a tray 26 of elongate, generally rectangular configuration containing longitudinally thereof recesses comprising a first recess 28, a second recess 30, a third recess 32 and a fourth recess 34. The first recess 28 is dimensioned to receive the deflated balloon and a furling device 36. The second recess 30 is dimensioned to receive a holding sheath 38, the third recess 32 is designed to receive the coupling 16 and the fourth recess is designed to receive the syringe 18. The second and third recesses 30 and 32 in combination are long enough to accommodate the catheter tube 12.

The furling device 36 as shown in FIGS. 1, 4, 5 and 6 is an elongate structure of generally rectangular configuration comprised of two parts, a bottom part 40, a top part 42 and connectors 44-44 for detachably securing them to each other. The bottom part 40 comprises an elongate block 46 having top and bottom surfaces 48 and 50 and spaced, parallel, longitudinal edge flanges 52-52. The top surface 48 contains longitudinally thereof a shallow recess 58 of a width corresponding substantially to the width of a deflated balloon and a depth greater than the thickness of the deflated balloon. A groove 60 extends longitudinally of the recess midway between its opposite sides of a cross section corresponding substantially to the desired diameter or cross section of the furled balloon. Immediately adjacent the groove 60 are two longitudinally-raised portions 61-61 which are configured so that the distance between the raised portions and top part 42 is slightly greater than the thickness of the deflated balloon, but less than the thickness of the wire 14 plus the deflated balloon. These raised portions insure that the wire 14 remains in groove 60 during furling and that the deflated balloon furls smoothly about the wire. The top part 42 comprises a flat plate corresponding in length and width to the bottom part. The top and bottom parts are removably held engaged by the connectors 44-44 which are slidably engaged with the flanges 52-52 and the opposite edges of the top part 42. In an alternative embodiment, block 46 can be formed to include connectors 44-44 as an integral part thereof. The furling device as thus related is dimensioned to be non-rotatably received in the recess 28 and to hold the balloon in a flattened, noncompressed condition.

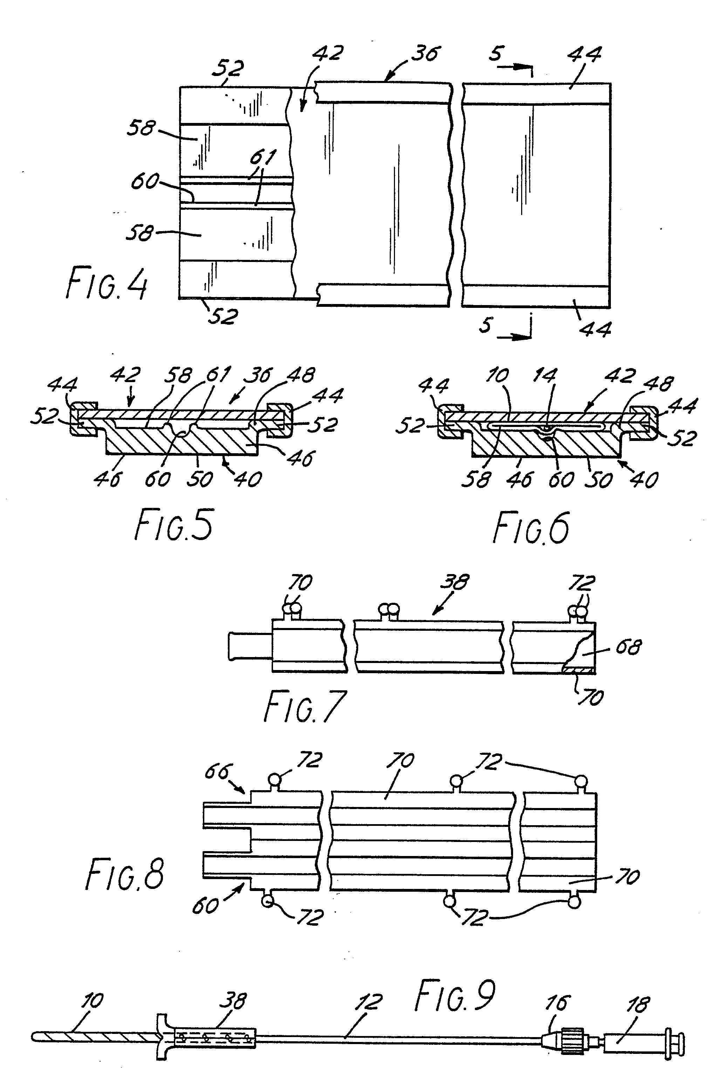

In the embodiment illustrated, the holding sheath 38, FIGS. 7 and 8, is in the form of a split tube comprising two parts 66-66 disposed in adjacency such as to define a longitudinal passage 68 of a diameter to receive the furled balloon. There are flanges 70-70 at the opposite longitudinal edges of the parts, two of which at one side are hinged to each other. There are latches 72-72 at the other two edges for releasably holding the two parts closed. The holding sheath 38 fits within the second recess 30 with one end adjacent the first recess 28 and the other end adjacent the third recess 32. Alternatively, other forms of a sheath, such as, for example, a splittable sheath, can be used. A splittable sheath as a tube having one or more pre-scored lines along the longitudinal dimension so that the sheath can be easily split and removed after its function has been performed.

Preferably, the sheath is at least long enough to cover one end and the mid-section of the furled balloon to prevent the balloon from unfurling prior to insertion into the patient.

The third recess 32, as previously mentioned, is long enough and deep enough to accommodate the catheter 12. The fourth recess 34 is of a length and depth to receive the coupling 16 and the syringe 18.

Peripherally of the aforesaid recesses is an elongate recess 35, FIG. 3, comprising spaced, parallel lengths extending longitudinally of the tray, the ends of which are joined by half-circular lengths for conveniently receiving a tube 22 for connecting the IAB device to a diastolic pump.

The balloon, catheter, coupling, syringe and tube are disposed in the tray 26 and a cover sheet 74 is applied to the top side to form a sealed package. As thus packaged, the balloon is deflated and loosely enough contained in the furling fixture 36 so that there is no possibility of the walls of the balloon becoming creased and/or adhered to each other.

When ready for use, the cover sheet 74 is removed, the balloon is evacuated by attaching the syringe 18 to the coupling 16 and pulling back on the plunger to create a vacuum, whereupon the syringe 18 is detached and the coupling 16 grasped and rotated. Alternatively, a vacutainer or other means can be used to evacuate he balloon prior to furling. Since one end of the deflated balloon 10 is fixed to the wire 14 and the other end to the catheter 12, rotation of the wire and catheter in unison relative to the furling device 36 which is held stationary furls the balloon. In contrast to prior practice and devices for furling balloons, the furling extends from the mid-length of the balloon toward its opposite ends rather than from one end to the other, as illustrated in FIG. 2, thus insuring uniform, non-kinking twist throughout its length and insuring complete unfurling when the balloon is inserted into a patient. When the furling is completed, the furled balloon is withdrawn from the furling device 36 longitudinally into the open end of the holding sheath 38 to an extent to constrain approximately half of the length of the furled balloon, FIG. 9, whereupon the furled balloon is inserted and, as it is moved into place, the holding sheath 38 is removed from the balloon and subsequently preferably removed from the catheter.

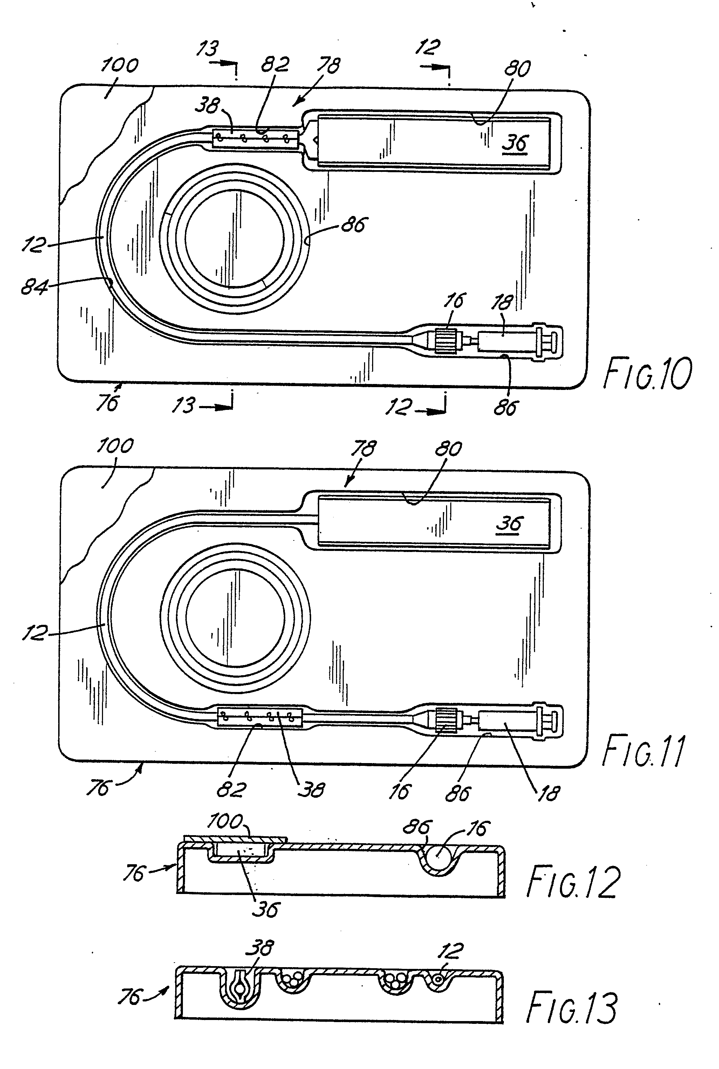

The package just described and illustrated in FIG. 3 has one disadvantage in that it is somewhat ungainly because of its length and, hence, not convenient for limited storage space. To this end, a second form of package is provided, FIGS. 10 to 13, of approximately half the length and twice the width of the first package and again is in the form of a tray. The tray 76 as shown in FIGS. 10 to 13 has generally spaced, parallel sides and ends. Recesses 80, 82, 84 and 86 extend continuously from the one end along one side around the other end and back along the other side. The recess 80 is of generally rectangular configuration corresponding substantially in length and depth to the furling device 36. The recess 82 is dimensioned to receive the holding sheath 38. The recesses 84 and 86 are dimensioned to receivethe catheter 12, coupling 16 and syringe 18. Alternatively, as shown in FIG. 11, the recess 82 can be located at the opposite side from the recess 80 in line with the recess 86.

The balloon 10 is positioned in the first recess 80 in a deflated condition enclosed within the furling device 36. The sheath 38 is positioned in the recess 82 and the catheter tube, valve and pump in the remaining recesses. A cover sheet 100 is applied over the top of the tray. As thus packaged, the balloon is deflated and held in an uncompressed condition.

When ready for use, the cover sheet 100 is stripped from the tray and the syringe 18 is connected to the coupling 16 to withdraw air from the balloon, whereupon the coupling 16 is rotated to furl the balloon. Rotation of the coupling 16 rotates the ends of the balloon relative to the portion thereof between the ends. Furling is continued until the furled balloon seats in the groove 60, whereupon the furled balloon is drawn into the sheath 38 to at least its mid-length, FIG. 9. The furled balloon can then be inserted and connected in the usual manner by way of a flexible tube to a diastolic pump. The flexible tube can be conveniently stored in recess 86, FIG. 10. The coupling 16, as aforesaid, preferably embodies a check valve so that when the syringe 18 is detached, the vacuum will be maintained in the balloon.

The trays 26,73 thus described are formed of a flexible plastic and, desirably, at least the cover is comprised of transparent plastic so as to enable easily assessing the kind or content of apparatus sealed therein.

The apparatus and method for furling balloon catheters described above lessen the likelihood of over-wrap encountered with prior art mechanically wrapped balloon catheters. The use of furled balloons in accordance with the present invention helps to reduce questionable unwrapping of the balloon once inserted into the patient. In addition, balloons furled in accordance with this invention can have a carefully controlled diameter to pass through a smaller percutaneous introducer sheath, thereby permitting improved blood flow. In accordance with this invention. The balloon should not be over-furled because the balloon will rotate freely within the groove of the furling device.

It should be understood that the description above is for the purpose of illustration only.

For instance, although a single lumen balloon catheter is described, the invention also includes furling the balloon of double lumen balloon catheters which can be accomplished in a similar manner.

## Claims

1. Structure for furling a deflated, generally flat balloon of a balloon catheter and confining at least a portion thereof within a sheath preparatory to use, said structure comprising relatively rotatable means engaged with the ends of the deflated balloon and with the balloon intermediate its ends, said means being relatively rotatable to furl the balloon about its longitudinal axis in opposite directions with respect to its mid-length to contract the deflated balloon to a less than predetermined diameter from end-to-end, a sheath defining an elongate tubular passage corresponding in internal diameter to the outside diameter of the furled balloon, said sheath supported in axial alignment with the furled balloon, and means for effecting movement of a portion at least of the furled balloon into said sheath to constrain the furled balloon in said furled condition.

2. Structure for furling a deflated balloon according to claim 1 comprising means for evacuating the deflated balloon prior to furling.

3. Structure for furling a deflated balloon according to claim 1 wherein said relatively rotatable means comprise a catheter connected to the proximal end of the balloon, a wire extending through the balloon to the distal end and connected thereto, and a friction-engendering device to engage the balloon intermediate its ends as the balloon is furled from both ends toward its mid-section to furl the balloon in a substantially uniform manner.

4. Means for furling a deflated balloon according to claim 3 wherein said friction-engendering device comprises diametrically-arranged surfaces with which the portions of the deflated balloon at opposite sides of its longitudinal axis are maintained flat, said surfaces being relatively rotatable about the longitudinal axis of the deflated balloon and operable by such rotation to furl the balloon from its ends towards its mid-section to a substantially uniform diameter throughout its length.

5. Structure for furling a deflated balloon according to claim 3 wherein said friction-engendering device comprises spaced, parallel surfaces corresponding substantially in width to the width of the deflated balloon and in spacing to substantially the thickness of the deflated balloon and wherein one at least of said surfaces has medially thereof a groove of a transverse width to receive the furled balloon when it has been constricted to its desired diameter.

6. Structure for furling a deflated balloon according to claim 3 wherein said friction-engendering device comprises an elongate block containing a recess longitudinally thereof corresponding in width and depth to the width and thickness of the deflated balloon and medially thereof a groove of a transverse width and depth corresponding to the desired diameter of the furled balloon and a cover plate secured to said block bridging the recess and the groove.

7. Structure for furling a deflated balloon according to claim 1 wherein the means engaged with the ends of the deflated balloon are, respectively, a catheter at the proximal end and a wire connected to the distal end.

8. Structure for furling a deflated balloon according to claim 1 wherein the means for effecting rotation comprises a coupling to which the distal end of the catheter and the distal end of the wire are connected.

9. Structure for furling a deflated balloon according to claim 1 wherein the means for evacuating the balloon comprises a syringe connected by way of a catheter to the balloon.

10. Structure for furling a deflated balloon according to claim 9 wherein the syringe is connected to the catheter by a coupling comprising a one-way valve.

11. A packaged, deflated balloon catheter, including a catheter connected at its distal end to the proximal end of a balloon, a check valve connected to the proximal end of the catheter and a wire connected at its proximal end to the proximal end of the catheter end and at its distal end to the distal end of the balloon, said package comprising a tray containing a recess for receiving the deflated balloon, catheter and check valve, a furling device non-rotatably disposed in the recess about the deflated balloon and a holding sheath disposed in the recess adjacent the furling device, said catheter and wire being relatively rotatable with respect to the mid-section of said balloon and to the furling device to furl the balloon within the furling device from its ends towards its mid-section.

12. A packaged, deflated balloon catheter according to claim 11 wherein said check valve and catheter are movable along said recess to retract at least a portion of the furled balloon into the holding sheath.

13. A packaged, deflated balloon catheter according to claim 12 comprising a syringe disposed in the recess connectable to the check valve by means of which the balloon can be evacuated prior to furling.

14. A packaged, deflated balloon catheter according to claim 13 wherein a cover sheet coextensive in length and breadth is sealably applied to the tray to hermetically seal the balloon, catheter, check valve and syringe, together with the furling device and sheath in the tray.

15. A furling fixture for furling a deflated balloon of a balloon catheter, said fixture comprising an elongate retaining structure defining a recess corresponding substantially in width to the width of the deflated balloon and in minimum depth to substantially the thickness of the deflated balloon, said fixture being operable when rotated relative to the ends of the balloon catheter to furl the balloon from its mid-length to its ends to no greater than a predetermined diameter from end-to-end.

16. A furling fixture for furling a deflated balloon of a balloon catheter comprising an elongate retaining structure defining a recess corresponding substantially in width to the width of the deflated balloon and in minimum depth to substantially the thickness of the deflated balloon, said fixture being operable when the ends of the balloon are rotated relative thereto to furl the balloon from its mid-length to its ends to no greater than a predetermined diameter from end-to-end.

17. A furling fixture according to claim 15 wherein said recess contains a longitudinally-extending groove from one end to the other located midway between its sides for retaining the furled balloon having a predetermined diameter.

18. A furling fixture according to claim 15 wherein said structure comprises facing bottom and top parts substantially coextensive in length and width and the bottom part contains a groove.

19. A furling fixture according to claim 18 wherein there are clamps engaged with the opposite edges of the bottom and top parts for holding the bottom and top parts detachably engaged.

20. A furling fixture according to claim 18 wherein the bottom part comprises means for engaging and retaining the top part in fixed relation thereto.

21. A furling fixture according to claim 18 wherein the groove is bounded along its opposite sides by ribs of a lesser height than the depth of the remaining portions of the recess.

22. A furling fixture for furling a deflated balloon of a balloon catheter comprising means for retaining the opposite broad faces of the deflated balloon in a substantially flat position at opposite sides of the longitudinal axis, said means being operable to effect rotation of the balloon at opposite sides of its longitudinal axis relative to its longitudinal axis and simultaneously to relinquish the balloon at opposite sides of its longitudinal axis for constriction there to a furled diameter of substantially uniform cross section from end-to-end.

23. A method of preparing a balloon catheter for insertion into a patient, said method comprising evacuating the balloon to cause it to become flattened throughout its length from end-to-end and to eliminate air pockets, furling the flattened balloon about its longitudinal axis commencing at its mid-length and proceeding to its opposite ends to condense the balloon to a predetermined diameter from end-to-end and confining the furled, condensed balloon within a tubular sheath of an inside diameter corresponding to substantially the predetermined diameter of the furled balloon, wherein said furling step is accomplished prior to removing the balloon catheter from a tray by using a furling fixture disposed within said tray, said fixtures comprising means for retaining the opposite broad faces of the deflated balloon in a substantially flat position at opposite sides of the longitudinal axis, said means being operable to effect rotation of the balloon at opposite sides of its longitudinal axis relative to its longitudinal axis and simultaneously to relinquish the balloon at opposite sides of its longitudinal axis for constriction there to a furled diameter of substantially uniform cross section from end-to-end.

24. The method of providing a balloon catheter for use, said method comprising providing the balloon in a deflated condition in an encapsulated receptacle together with a furling device disposed about the balloon and a tubular sheath for receiving the furled balloon, opening the receptacle, effecting rotation of the ends of the balloon relative to the furling device to impart twist to the balloon in opposite directions from its mid-length to its opposite ends and to constrict it to a diameter to be received within the sheath, drawing the furled balloon into the sheath and removing the sheathed furled balloon from the receptacle.

25. The method according to claim 24 comprising evacuating the balloon prior to furling.

26. The method according to claim 24 wherein a catheter is connected to the proximal end of the balloon, a wire extending longitudinally through the catheter and the balloon is connected to the distal end of the balloon and the catheter and wire are rotated in unison to effect furling of the balloon from its ends towards its mid-length.

FIG.1

FIG.2

FIG.3

FIG.3A

FIG.3B

FIG.3C

FIG.3C

0 256 683

FIG.4

FIG.5

FIG.6

FIG.7

FIG.8

FIG.9

FIG.10

FIG.11

FIG.12

FIG.13